# EUROPÄISCHE PATENTSCHRIFT

(11) **EP 3 282 847 B1**
(45) Veröffentlichungstag und Bekanntmachung des Hinweises auf die Patenterteilung: **08.07.2020**
(21) Anmeldenummer: 16727286.3
(22) Anmeldetag: 13.04.2016
(51) Int. Cl.: A01N 63/00, C12R 1/07, C12N 11/00, C12N 1/20, C05F 11/02, C05G 3/00

(54) **SELEKTION UND EINSATZ KÄLTE-TOLERANTER BACILLUSSTÄMME ALS BIOLOGISCHE PHYTOSTIMULATOREN**
SELECTION AND USE OF COLD-TOLERANT BACILLUS STRAINS AS BIOLOGICAL PHYTOSTIMULATORS
SÉLECTION ET UTILISATION DE SOUCHES DE BACILLUS RÉSISTANT AU FROID EN TANT QUE PHYTOSTIMULANTS BIOLOGIQUES

(30) Priorität: 14.04.2015 DE 102015004809
(43) Veröffentlichungstag der Anmeldung: 21.02.2018
(73) Patentinhaber: Abitep GmbH, 12489 Berlin (DE)
(72) Erfinder: BORRISS, Rainer, 17489 Greifswald (DE); DIETEL, Kristin, 10783 Berlin (DE); BEIFORT, Paul, 12487 Berlin (DE)
(74) Vertreter: Baumbach, Friedrich
(86) Internationale Anmeldenummer: PCT/DE2016/000159
(87) Internationale Veröffentlichungsnummer: WO 2016/165685

(56) Entgegenhaltungen:
- GULATI, A.; VYAS, P.; RAI, P.; KASANA, R.C.: "Plant growth promoting and rhizosphere-competent Acinetobacter rhizosphaerae strain BIHB 723 from the cold deserts of the Himalayas.", CURR.MICROBIOL., Bd. 58, 2009, Seiten 371-377, XP19708342, in der Anmeldung erwähnt
- YADAV AJAR NATH ET AL: "Culturable diversity and functional annotation of psychrotrophic bacteria from cold desert of Leh Ladakh (India)", WORLD JOURNAL OF MICROBIOLOGY AND BIOTECHNOLOGY, RAPID COMMUNICATIONS OF OXFORD, OXFORD, GB, Bd. 31, Nr. 1, 5. November 2014 (2014-11-05), Seiten 95-108, XP035418560, ISSN: 0959-3993, DOI: 10.1007/S11274-014-1768-Z [gefunden am 2014-11-05]
- YADAV AJAR NATH ET AL: "Prospecting cold deserts of north western Himalayas for microbial diversity and plant growth promoting attributes", JOURNAL OF BIOSCIENCE AND BIOENGINEERING, ELSEVIER, AMSTERDAM, NL, Bd. 119, Nr. 6, 6. Januar 2015 (2015-01-06), Seiten 683-693, XP029586225, ISSN: 1389-1723, DOI: 10.1016/J.JBIOSC.2014.11.006
- Ajar Nath Yadav ET AL: "Bioprospecting of plant growth promoting psychrotrophic Bacilli from the cold desert of north western Indian Himalayas", Indian journal of experimental biology, 1. Februar 2016 (2016-02-01), Seiten 142-150, XP55295872, India Gefunden im Internet: URL:http://nopr.niscair.res.in/bitstream/1 23456789/33745/1/IJEB 54(2) 142-150.pdf

### SEQUENCE LISTING

<110> ABiTEP GmbH
<120> Selektion und Einsatz kälte-toleranter Bacillusstämme als biologische Phytostimulatoren
<130> Abitep 2 EP
<140> EP16727286.3
   <141> 2016-04-13
<160> 6
<170> PatentIn version 3.5
<210> 1
   <211> 27
   <212> DNA
   <213> Artificial Sequence
<220>
   <223> Primer pRB1601
<220>
   <221> misc-feature
   <223> pRB1601
<400> 1
   ggatcctaat acatgcaagt cgagcgg 27
<210> 2
   <211> 28
   <212> DNA
   <213> Artificial Sequence
<220>
   <223> Primer pRB1602
<220>
   <221> misc-feature
   <223> pRB1602
<400> 2
   ggatccacgt attaccgcgg ctgctggc 28
<210> 3
   <211> 21
   <212> DNA
   <213> Artificial Sequence
<220>
   <223> Primer gyrFW
<220>
   <221> misc-feature
   <223> gyrFW
<400> 3
   cagtcaggaa atgcgtacgt c 21
<210> 4
   <211> 21
   <212> DNA
   <213> Artificial Sequence
<220>
   <223> Primer gyrRV
<220>
   <221> misc-feature
   <223> gyrRV
<400> 4
   caaggtaatg ctccaggcat t 21
<210> 5
   <211> 35
   <212> DNA
   <213> Artificial Sequence
<220>
   <223> Primer cheAFW
<220>
   <221> misc-feature
   <223> cheAFW
<400> 5
   gaaacggaka yatggmagtb acmtcaract ggctg 35
<210> 6
   <211> 35
   <212> DNA
   <213> Artificial Sequence
<220>
   <223> Primer cheARV
<220>
   <221> misc-feature
   <223> cheARV
<400> 6
   tgctcragac gcccgcggwc aatgacaagc tcttc 35

## Beschreibung

Die Erfindung betrifft ein biologisches Mittel zur Ertragsverbesserung von Kulturpflanzen. Anwendungsgebiete der Erfindung sind Landwirtschaft, Gartenbau und Pflanzenschutz.

### Stand der Technik

Die Einführung von biologischen Pflanzenschutzmitteln bzw. Biodüngern stellt eine umweltfreundliche Alternative zu chemischen Pestiziden und mineralischen Düngern dar. "Plant-growth-promoting" Rhizobakterien (PGPR) werden für die Herstellung von wirksamen Bioformulierungen erfolgreich eingesetzt. Repräsentanten der Gattung *Bacillus* mit der Fähigkeit zur Bildung von dauerhaften Endosporen sind von großer Bedeutung, da sie eine natürliche hohe Stabilität aufweisen. Sporen sind Dauerformen der Bakterienzelle, die extremen Umwelteinflüssen wie Hitze und Trockenheit widerstehen und jahrelang ohne Aktivitätsverlust gelagert werden können.

Bioformulierungen von Repräsentanten der Gattung *Bacillus* sind die am häufigsten eingesetzten biologischen Pflanzenschutzmittel und Phytostimulatoren (Borriss 2011). Neben den bekannten Produzenten von Insektiziden *Bacillus thuringiensis* und *Bacillus kurstakii*, sind es Stämme aus dem Verwandtschaftskreis von *Bacillus subtilis*, u.a. *B. amyloliquefaciens* subsp. *plantarum, B. subtilis, B. licheniformis* und *B. pumilus*, die die meiste Anwendung auf diesem Gebiet erfahren haben. Beispiele für kommerzielle Produkte (Biodünger und/oder Pflanzenschutzmittel), die *B*. *amyloliquefaciens plantarum* Sporen enthalten, sind Kodiak™ (Bayer Crop Science), Companion (Growth Products Ltd.), BioYield™ (Bayer Crop Science), INTEGRAL® (BASF), VAULT® (BASF), SERENADE Max ® (Bayer Crop Science), CEASE^{(R)} (BioWorks, Inc.), RhizoVital® (ABiTEP GmbH), FZB24® (ABiTEP GmbH), Double Nickel 55™ (Certis U.S.A.), Amylo-X® (Certis U.S.A.). Pflanzenschutzmittel auf der Basis von *B. licheniformis* sind Green Releaf and EcoGuard (Novozyme Biologicals Inc.). Der Stamm *B. pumilus* GB34 (Yield Shield, Bayer Crop Science) ist aktiver Bestandteil biologischer Fungizide. Andere EPA registrierte *B. pumilus* Biofungizide sind SONATA (Bayer Crop Science), und GHA 180 (Premier Horticulture). Vertreter von *B*. *mojavensis* wurden ebenfalls als phytostimulatorisch und fähig zu endophytischem Wachstum beschrieben (Bacon & Hinton 2002). Andere Bacillen, die nicht dem *B. subtilis* Species Komplex angehören, stimulieren ebenfalls das Pflanzenwachstum und die Pflanzengesundheit. *B. firmus* GB126 (BioNem AgroGreen, Israel jetzt übernommen von Bayer Crop Science) wird als EPAregistriertes Nematicid zur biologischen Kontrolle von phytopathogenen Nematoden eingesetzt. Ein ähnliches Produkt von *B. firmus* Bmj WG wird gegenwärtig von Certis U.S.A. entwickelt. Ein phytostimulatorischer *B*. *megaterium* (López-Bucio et al. 2007) Stamm ist Grundlage des Biofungizids BioArc. Vertreter des *Bacillus cereus* Species Komplex, z.B. *B. cereus* UW8 (Handelsman et al. 1990), *B*. *thuringiensis* und *B*. *weihenstephanensis* wurden ebenfalls als pflanzenwachstumsfördernde, endosporen-bildende Rhizobakterien beschrieben.

Diese für die Herstellung der gegenwärtigen Bioprodukte eingesetzten Bakterien sind als "mesophile" Bakterien bekannt und benötigen Wachstumstemperaturen von mehr als 15°C, um ihre Wirkung als Phytostimulatoren zu entfalten. Mehr als 85% der Erdoberfläche werden jedoch durch kalte Ökosysteme, einschließlich alpine Bergregionen, polarnahe und ozeanische Gebiete, eingenommen (Feller & Gerday 2003). Bei Temperaturbedingungen unter 15°C, wie sie für diese Regionen typisch sind, können mesophile Bakterien keine positive Wirkung auf das Pflanzenwachstum ausüben. Eine attraktive Alternative stellt der Einsatz kälte-toleranter Mikroorganismen, dar. Obwohl ihr Wachstumsoptimum im mesophilen Bereich liegt, haben diese Mikroorganismen Mechanismen entwickelt, die ihnen die Fähigkeit verleiht, noch bei tiefen Temperaturen (≤10° - 0° C) zu wachsen (Kumar et al. 2010). Es wurde darauf hingewiesen, dass der Einsatz von kälte-toleranten und pflanzenwachstumsfördernden Bakterien als Inokulantien insbesondere für die Agrarproduktion in temperierten Klimazonen mit niedrigen Temperaturen und kurzer Wachstumssaison attraktiv sein kann (Mishra et al. 2012). Allerdings beschränken sich die bekannten Beispiele für den Einsatz kälte-toleranter Bakterien im Pflanzenbau auf Gram-negative phytostimulatorische Bakterien, wie *Pseudomonas* (Katiyar & Goel 2003), *Burkholderia* (Barka et al. 2006), *Acinetobacter* (Gulati et al. 2009), *Azospirillum* (Kaushik et al. 2002), *Rhizobium* (Prevost et al. 2003), *Bradyrhizobium* (Zhang et al. 2003), *Pantoea* (Selvakumar et al. 2008a), *Serratia* (Selvakumar et al. 2008b). Obwohl in der Vergangenheit einige psychrophile bzw. kälte-tolerante *Bacillus-Arten*, wie *Bacillus globisporus* (jetzt *Sporosarcina globisporus*), *Bacillus insolitus* (jetzt: *Psychrobacillus insolitus*), *Paenibacillus macquariensis, Bacillus marinus* (jetzt: *Marinibacillus marinus*), *Bacillus psychrophilus* (jetzt: *Sporosarcina psychrophilus*)*, Bacillus psychrosaccharolyticus, Bacillus psychrotolerans* (jetzt: *Psychrobacillus psychrotolerans*) und *Bacillus psychrodurans* (jetzt: *Psychrobacillus psychrodurans*) beschrieben wurden (El-Rahman et al. 2002, Krishnamurti et al. 2010) fehlen Informationen über kältetolerante *Bacillus* Stämme mit phytostimulatorischer Wirkung.

In Yadav Ajar Nath et al. World journal of microbiology and Biotechnology, Rapid communications of Oxford, Oxford GB, Bd 31, Nr. 1, 5.November 2014, Seiten 95-108 wird offenbart, dass der kältetolerante Stamm Bacillus simplex IARI-L-118 (s. Tabelle 2) das Pflanzenwachstum fördert.

In Yadav Ajar Nath et al. Journal of Bioscience and Bioengineering, Elsevier, Amsterdam, NL, Bd. 119, Nr. 6, 6. Januar 2015 (2015-01-06), Seiten 683-693 wird offenbart, dass der kältetolerante Stamm Bacillus simplex IARI-R-3 (s. Tabelle 2) das Pflanzenwachstum fördert.

### Ziel und Aufgabe der Erfindung

Die Erfindung hat das Ziel, ein umweltverträgliches und nachhaltiges Mittel zur Stimulierung des Pflanzenwachstums auch bei Temperaturen unterhalb von 15°C zu entwickeln. Das Mittel soll insbesondere in klimatisch ungünstigen Regionen, z.B. Bergregionen und relativ Pol-nahen Gebieten der nördlichen und südlichen Hemisphäre, zum Einsatz kommen.

### Wesen der Erfindung

Die Aufgabe wird gemäß Anspruch gelöst, die Unteransprüche 2 - 9 sind Vorzugsvarianten.

Kern der Erfindung ist der Einsatz kälte-toleranter Bacillus-Stämme als biologische Phytostimulatoren. Zum Umfang der Erfindung gehört weiterhin die Kombination der kälte-toleranten Bacillus-Stämme mit mesophilen Bakterien der Ordnung Bacillales bevorzugt der Gattung Bacillus und Paenibacillus und die Kombination mit Huminsäuren. Bei der Suche nach Pflanzen-wachstumsfördernden Bakterien, die bei Temperaturen unter 15°C ein eindeutiges Wachstum zeigen, wurde gefunden, dass Endosporen-bildende Bacillen, die aus kühlen Klimaregionen, z. B. Bergregionen dem Hochland von Tibet (VR China) oder den Voralpen(1400 m), isoliert wurden, auch bei Temperaturen unterhalb von 12°C zu wachsen vermögen. Entsprechend ihrer 16S rRNA Sequenzen wurden diese kälte-adaptierten Bakterien den Arten *Bacillus simplex*, *Bacillus pumilus*, sowie dem *Bacillus subtilis* Species Komplex zugeordnet. Aufgrund ihrer *gyrA* und *cheA* Sequenzen wurden die Vertreter des *B*. *subtilis* Species-Komplex als zugehörig zur Art *Bacillus atrophaeus* identifiziert. Die pflanzenwachstumsfördernde Wirkung wurde mit *Arabidopsis-* Keimlingen in Topfexperimenten untersucht. Kälte-tolerante Vertreter der Arten *Bacillus simplex, Bacillus atrophaeus* und *Bacillus pumilus* zeigten eine deutliche phytostimulatorische Wirkung (Beispiel 6). Die Stämme *B*. *simplex* (ABI02S), *B. atrophaeus* (ABI02A), und *B. pumilus* (ABI02P1) wurden als exemplarisch für die weiteren Untersuchungen ausgewählt. Nachfolgend eine Beschreibung dieser Stämme:
*Bacillus atrophaeus* unterscheidet sich von *B*. *subtilis* durch seine DNA-Sequenz und die Bildung eines dunklen Pigments (Nakamura 1989). Seine Fähigkeit zur Förderung des Pflanzenwachstums wurde dokumentiert (Karlidag et al. 2010, Ertuk et al. 2011, Chan et al. 2013). Die taxonomische Zugehörigkeit des Stammes ABI02A zu *Bacillus atrophaeus* wurde durch Vergleich seiner 16S rRNA, der gyrA und der cheA Partialsequenz bestimmt (siehe Beispiel 1):

**Tabelle 1**

| Primer | Genfragment | Species | Akzession | BLAST |
|---|---|---|---|---|
| pRB1601 | 16SrRNA | *B*. *atrophaeus* 1942 | CP002207.1 | 451/453 (99%) |
| pRB1602 | 16SrRNA | *B*. *atrophaeus* 1942 | CP002207.1 | 470/473 (99%) |
| gyrFW | gyrA | *B*. *atrophaeus* 1942 | CP002207.1 | 863/872 (99%) |
| gyrRV | gyrA | *B*. *atrophaeus* 1942 | CP002207.1 | 857/865 (99%) |
| cheAFW | cheA | *B*. *atrophaeus* 1942 | CP002207.1 | 604/698 (87%) |
| cheARV | cheA | *B*. *atrophaeus* 1942 | CP002207.1 | 509/533 (95%) |

ABI02A, ABI03 und ABI05 sind durch die folgenden Eigenschaften charakterisiert: Die physiologischen Eigenschaften von ABI02A, ABI03 und ABI05 sind im Anwendungsbeispiel 3 dargestellt. Die Stämme fördern das Pflanzenwachstum (siehe Anwendungsbeispiel 1), supprimieren das Wachstum von bakteriellen (*Xanthomonas oryzae*) und pilzlichen (*Sclerotinia sclerotorum*) Pathogenen *in vitro.* ABI02A wurde als Stamm DSM 32019, ABI03 wurde als Stamm DSM 32285 und ABI05 als Stamm DSM 24918 bei der DSMZ, Braunschweig hinterlegt. *Bacillus atrophaeus* ist bereits als pflanzenwachstumsförderndes Bakterium beschrieben worden (Bai et al. 2002). Über eine phytostimulatorische Wirkung bei niedrigen Temperaturen wurde jedoch wie bei *B. simplex* bisher nicht berichtet.

*Bacillus simplex* ABI02S und ABI12 wachsen bereits bei 4°C. Wachstumsmaximum ist 45°C. Die physiologischen Eigenschaften des Stammes sind im Ausführungsbeispiel 4 dargestellt. Vertreter der Species *Bacillus simplex* wurden gegenwärtig als phytostimulatorische Bakterien identifiziert (Schwartz et al. 2013). Über eine wachstumsstimulierende Wirkung bei niedrigen Temperaturen wurde bisher nicht berichtet. Der Stamm ABI02S wurde als DSM 32020 und der Stamm ABI12 als DSM 32283 bei der DSMZ Braunschweig, Deutschland hinterlegt.

Die Erfindung wird nachfolgend an Beispielen näher erläutert.

### Ausführungsbeispiele

### Beispiel 1: Isolierung von potentiell kälte-toleranten Bacillen

Kälte-tolerante Bacillus-Stämme wurden z.B. aus dem Hochland der Autonomen Provinz Tibet und den Voralpen, Höhe 1400 m isoliert. Das tibetanische Bergland liegt in einer durchschnittlichen Höhe von 4000 m und weist eine jährliche Durchschnittstemperatur von 10°C auf. Typisch für diese Region sind die großen Tag- und Nacht-Temperaturunterschiede. Die Proben wurden entweder direkt von Pflanzenwurzeln oder von der anhaftenden Erde entnommen:
2,5 g Probenmaterial wurden in 25 g destilliertem Wasser für 2 h unter kontinuierlichem Schütteln resuspendiert. Zur Abtötung vegetativer Zellen wurde die Suspension anschließend eine Stunde bei 80° C inkubiert. 10 ml der Suspension wurden zu 40 ml eines Mineral-Salz-Mediums gegeben und eine Woche bei 20° C inkubiert, bis bei mikroskopischer Kontrolle vegetative, stäbchen-förmige Zellen auftraten. Anschließend wurde die Suspension 10⁻¹ bis 10⁻⁵ verdünnt und auf Minimalagar ausplattiert. Die Platten wurden bei 20°C inkubiert und die gebildeten Kolonien auf Nährgar bzw. LB-Agar vereinzelt.

Die taxonomische Einordnung der Isolate wurde durch Bestimmung ihrer 16S rRNA-Sequenz, sowie bei Stämmen aus dem Verwandtschaftskreis von *B. subtilis* durch ihre *gyrA* und *cheA* Sequenz vorgenommen. Die Isolierung der chromosomalen DNA von exponentiell wachsenden *Bacillus*-Zellen, DNA-Amplifikation und Sequenzierung erfolgte nach Idriss et al. 2002. Dabei wurden die folgenden Primer für die Amplifikation der DNA-Sequenzen mittels Polymerase Ketten-Reaktion (PCR) eingesetzt:
pRB1601: 5'GGATCCTAATACATGCAAGTCGAGCGG
pRB1602: 5'GGATCCACGTATTACCGCGGCTGCTGGC
gyrFW: 5' CAGTCAGGAAATGCGTACGTC
gyrRV: 5' CAAGGTAATGCTCCAGGCATT
cheAFW: 5' GAAACGGAKAYATGGMAGTBACMTCARACTGGCTG
cheARV: 5' TGCTCRAGACGCCCGCGGWCAATGACAAGCTCTTC

Eine Übersicht über die erhaltenen Isolate und ihre taxonomische Einordnung auf der Basis ihrer 16S rRNA Sequenz ist in der Tabelle 2 gegeben.

**Tabelle 2: DNA Sequenzen von Kälte-adaptierten Bacillus Isolaten aus dem Tibetanischen Hochland und den Voralpen (Höhe 1400m)**

| Stamm | Ort der Isolierung | 16SrRNA-Ähnlichkeit | PGP¹ | Wachstum |
|---|---|---|---|---|
| ABI02S | Graswurzeln, Mount Sejila, Nyingtri, Tibet | *B.simplex* 338/358(94%) (pRB1601) *B.simplex* 403/464(87%) (pRB1602) | + | 04 - 45°C |
| ABI02A | Graswurzeln, Nyingtri, Tibet | *B. atropheus* 450/458 (98%) (pRB1601) *B.atrophaeus* 572/590(97%) (gyrFW) *B.atrophaeus* 733/757(97%) (gyrREV) | + | 10 - 45°C |
| ABI02P1 | Namtso See, Lhasa, Tibet | *B. pumilus* 457/468(98%) (pRB1602) | + | 10 - 50°C |
| ABI03 | Graswurzeln, Voralpen, Höhe 1400 m | *B. atropheus* 446/465 (96%) (pRB1601) 474/475 (99%) *B.atrophaeus* 608/625 (97%) (gyrFW) (pRB1602) | + | 10-45°C |
| ABI05 | Graswurzeln, Voralpen, Höhe 1400 m | *Bacillus atrophaeus* 450/458 (98%) (pRB1601) *447*/*478(94%)* (pRB1601) | + | 10-45°C |
| ABI12 | Graswurzeln, Voralpen, Höhe 1400 m) | *B. simplex* 450/472 (95%) (pRB1601) 461/472(98%) (pRB1602) | + | 04-45°C |

| | | | | |
|---|---|---|---|---|
| PGP = pflanzenwachstumsfördernde Wirkung. | | | | |

### Beispiel 2: Wachstumscharakteristik von Kälte-toleranten Bakterien

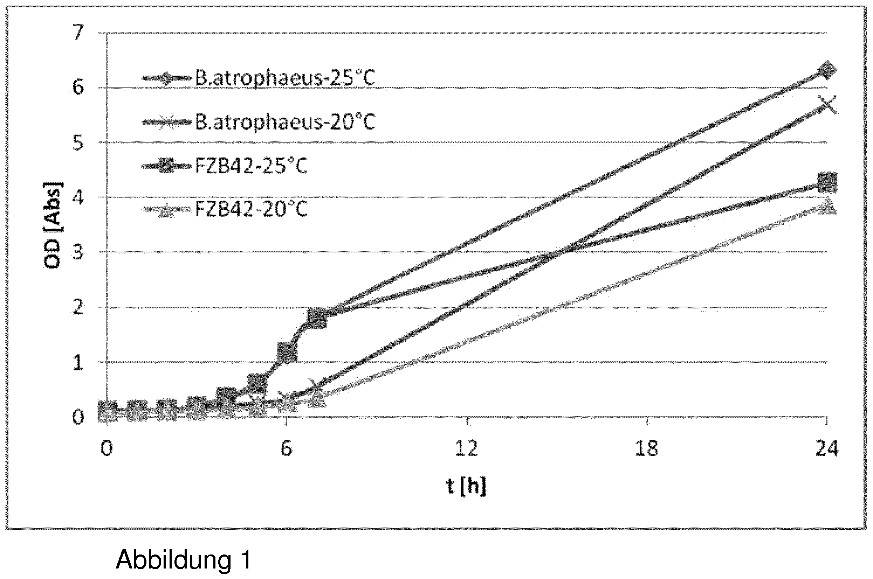

Die Wachstumsversuche wurden in LB-Medium bei unterschiedlichen Temperaturen durchgeführt. Die Tabelle 2 gibt eine Übersicht über das Wachstumsverhalten der Kälte-toleranten Stämme. Die Obergrenze für das Wachstum von *B. simplex* ABI02S und ABI12, sowie *B. atrophaeus* ABI02A ABI03 und ABI05 waren 45°C, während *B*. *pumilus* ABI02P1 auch bei 50°C wächst. *B. atrophaeus* und der mesophile *B*. *amyloliquefaciens* subsp. *plantarum* FZB42 wurden bei 20°C und 25° kultiviert. Bei diesen Temperaturen wies der Kälte-tolerante *B. atrophaeus* Stamm eine höhere Wachstums-Geschwindigkeit als FZB42 auf (Abb.1).

*B. atrophaeus* ABI02A wurde bei verschiedenen Temperaturen zwischen 20° bis 50°C kultiviert (Abb. 1). Das Wachstumsoptimum wurde bei 33 - 35°C bestimmt. Bei 30°C wurde eine leichte Verlangsamung der Wachstumsgeschwindigkeit registriert. Unterhalb von 30° C wurde dieser Effekt weiter verstärkt (25°C und 20°C). Bei 40° C war das Wachstum signifikant verzögert. Bei 50°C wurde zusätzlich eine Lysis der Zellen beobachtet. Diese Ergebnisse bestätigen, dass es sich im Falle von B. *atrophaeus* ABI02A um einen mesophilen, aber gleichzeitig kältetoleranten Stamm handelt, der trotz eines Wachstumsoptimums bei 33 - 35° C auch bei niedrigen Temperaturen noch gut wächst.

Die physiologischen Eigenschaften der kältetoleranten *Bacillus atrophaeus* und *Bacillus simplex* Stämme werden in den nachfolgenden Beispielen 3 und 4 näher beschrieben.

### Beispiel 3: Physiologische Eigenschaften von Bacillus atrophaeus

**Tabelle 3: Physiologische Eigenschaften von Bacillus atrophaeus ABI02A, ABI03 und ABI05**

| | |
|---|---|
| Verwertung von Zuckern | |
| Glucose | + |
| Saccharose | + |
| Lactose | - |
| Maltose | ± |
| Fructose | + |
| Voges Proskauer | + |
| Exoenzym-Bildung | |
| Lipase (Tributyrin) | + |
| Amylase (Stärke) | + |
| Protease (Casein) | + |
| Keratinase (Keratin) | - |
| Cellulase (Cellulose) | - |
| Eigelbtest (Lecithinase) | - |
| Pigmentbildung | **+** |
| Hemmung durch Antibiotika (Blättchentest) | |
| CM5 Chloramphenicol | **+** |
| KM5 Kanamycin | **+++** |
| ER1 Erythromycin | **++** |
| Li25 Lincomycin | **+** |
| AP100 Ampicillin | **++** |
| RIF 25** Rifampicin | **+++** |
| SPEC 100 Spectinomycin | **++** |
| Bleo1 Bleomycin | **+** |

Im Gegensatz zu *B. amyloliquefaciens* subsp. *plantarum* FZB42 wachsen *Bacillus atrophaeus* ABI02A, ABI03 und ABI05 auch bei 10°C. Bei 20°C und 25°C weist ABI02A eine höhere Wachstumsgeschwindigkeit als FZB42 auf . Im Agardiffusionstest inhibieren ABI02A, ABI03 und ABI05, *Bacillus subtilis, Bacillus megaterium*, sowie die phytopathogenen *Erwinia amylovora, Xanthomonas oryzae, Fusarium oxysporum, Rhizoctonia solani und Sclerotinia sclerotiorum.*

### Beispiel 4: Physiologische Eigenschaften von Bacillus simplex

*B. simplex* ABI02S und ABI12 haben die folgenden physiologischen Eigenschaften (Tabelle 4).

**Tabelle 4: Physiologische Eigenschaften von ABI02S und ABI12**

| | |
|---|---|
| Zellform | Stäbchen, 0.9-1.0 x 3.0->4.0 m |
| Endosporen | Ellipsoid |
| Sporenmutterzelle | Nicht geschwollen |
| Nutzung von Zitronensäure | + |
| Nutzung von Propionsäure | - |
| Katalase | + |
| Nitrate Reduktase (NO₂ von NO₃) | + |
| Phenylalanin desaminase | - |
| Arginin dihydrolase | - |
| Indol bildung | - |
| Anaerobes Wachstum | - |
| Voges Proskauer (Acetoinbildung) | - |
| pH in Voges Proskauer Medium | 6.2 |
| Lecithinase (Eigelbreaktion) | - |
| Wachstum bei 50°C | - |
| Wachstum bei 45°C | (+) |
| Wachstum bei 40°C | + |
| Wachstum bei pH 5.7 | - |
| Wachstum bei 2% NaCl | + |
| Wachstum bei 5% NaCl | - |
| Wachstum bei 7% NaCl | - |
| Wachstum bei 10% NaCl | - |
| Säurebildung von D-Glucose | + |
| Säurebildung von L-Arabinose | + |
| Säurebildung von D-Xylose | + |
| Säurebildung von D-Mannit | + |
| Säurebildung von D-Fructose | + |
| Gas von D-Glucose | - |
| Hydrolyse von Stärke | + |
| Hydrolyse von Gelatine | + |
| Hydrolyse von Casein | + |
| Hydrolyse von Tween 80 | + |
| Hydrolyse von Aesculin | (+) |

Die physiologischen Eigenschaften bestätigen die Zuordnung zu *Bacillus simplex* weitestgehend, sind aber nicht in allen Merkmalen typisch für *Bacillus Simplex.* Die Analyse der zellulären Fettsäuren zeigt ein für die Gattung *Bacillus* typisches Profil. *B. simplex* ABI02S und ABI12 wachsen in LB-Medium bei 10°C, einer Temperatur bei der *B*. *amyloliquefaciens* FZB42 nicht mehr zu wachsen vermag. In weiteren Experimenten zeigte sich, dass diese beiden *B. simplex* Stämme auch noch bei einer Temperatur von 4°C zu wachsen vermögen. Wachstumsmaximum war 45°C.

Im Agardiffusionstest hat *B*. *simplex* eine suppressive Wirkung auf die pathogenen Pilze *Rhizoctonia solani* und *Fusarium oxysporum,* sowie auf *Xanthomonas oryzae.*

### Beispiel 5: Fermentation von Bacillus atrophaeus ABI02A und Herstellung einer Sporensuspension

Beispielhaft erfolgt hier die Beschreibung für die Herstellung einer Sporensuspension für *B*. *atrophaeus* ABI02A. Die Fermentation von *B. atrophaeus* wurde in einem konventionellen Rührfermenter mit einem Ansatzvolumen von 1,4 L Medium unter den folgenden Bedingungen durchgeführt:
Medium: Vollmedium mit organischer N- und C-Quelle: Sojamehl bzw. Maisquellwasser, Magermilchpulver, Hefeextrakt, Salze
Sterilisation des Mediums: 20 min. bei 121 °C
Vorlage Antischaummittel: 200 mL
Rührerdrehzahl: 700 U/min.
Fermentationstemperatur: 33 °C
Belüftung: 0,7 L/min (40% O₂ Sättigung)
pH: mit NaOH auf 6,9 eingestellt

Nach 16 h wurde eine maximale Zelldichte von 1 x 10¹⁰ Zellen/mL erreicht. Die Kultur wurde weitergeführt, um eine möglichst vollständige Versporung der vegetativen Zellen zu erreichen. Nach 40 h wurde ein Sporentiter von 3 x 10⁹ erreicht. Die Sporen wurden abzentrifugiert und in 10% des ursprünglichen Mediumvolumens unter Zusatz von Propandiol (Endkonzentration: 5%) aufgenommen.

### Beispiel 6: Förderung der Keimung von Maissamen durch kälte-tolerante Bacillen

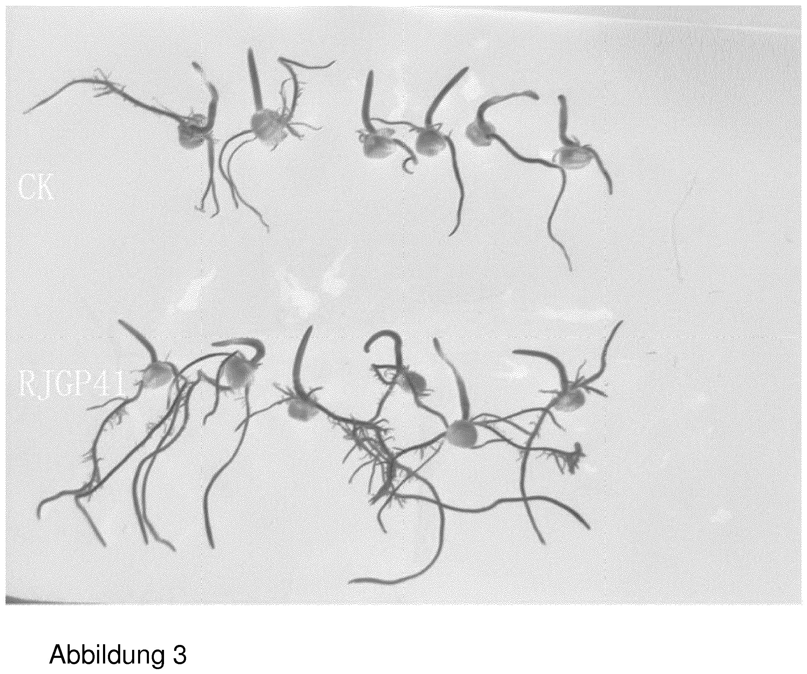

10 ml einer Bacillus-Sporensuspension wurden mit 30 ml 1% Carboxymethylcellulose (CMC), die als Adhäsiv für eine bessere Anhaftung der Sporen an der Maisoberfläche diente, gemischt. Die Endkonzentration der Bacillus-Sporen in der Suspension betrug 1 × 107, 1 ×106, 1 ×105, 1 ×104 cfu/ml. Die Maissamen wurden mit 75% Äthanol, 5% NaClO für 5 min. oberflächendesinfiziert, bevor sie mit den verschiedenen Verdünnungen der Bacillus-Sporensuspension für 5 min. behandelt wurden. Jeweils 3x 10 Samen einer Sporen-Konzentration wurden in einer Petrischale mit feuchtem Filterpapier ausgelegt. Die Bestimmung der Keimungsrate erfolgte nach einer Woche Dunkelinkubation bei 30°C. Als Kontrolle wurden Maiskerne verwendet, die mit sterilem Nährmedium + CMC behandelt worden waren. Eine deutliche Erhöhung der Keimungsgeschwindigkeit wurde bei Applikation von B. simplex und B. atrophaeus festgestellt (Abb. 3).

### Beispiel 7: Wachstums-Experimente mit Arabidopsis thaliana

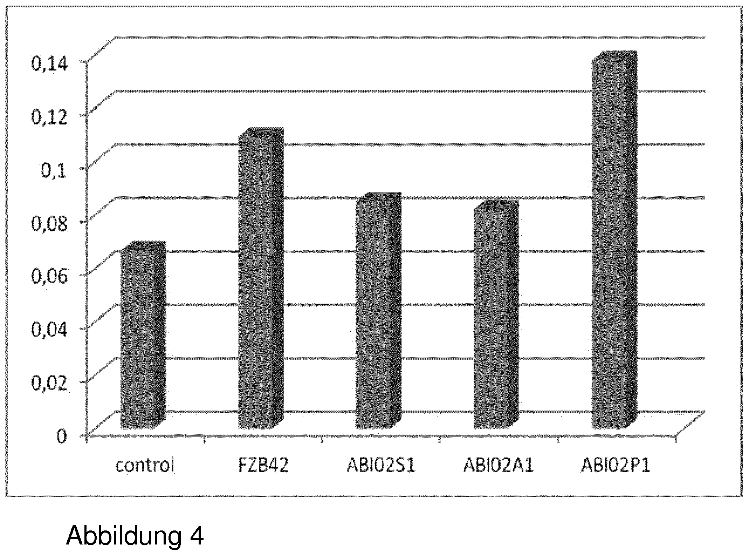

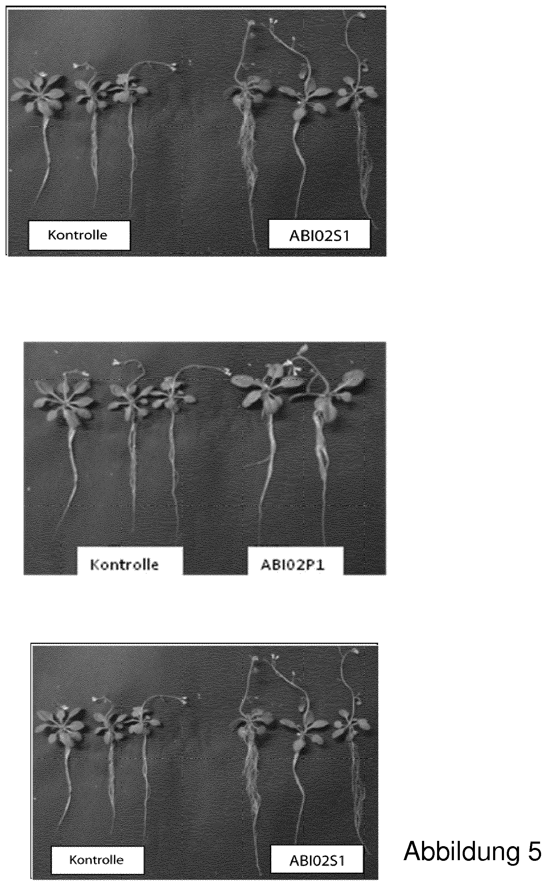

Die Wurzeln eines 6-Tage alten *Arabidopsis thaliana* Keimlings wurde für 5 min. in einer Sporensuspension (10⁵ Sporen/ml) kälte-adaptierter Bacillen für 5 min. inkubiert. Zum Vergleich wurde auch eine Behandlung mit einer Sporensuspension von FZB42, der für seine wachstumsfördernde Wirkung bekannt ist, durchgeführt. Anschließend wurden die behandelten Keimlinge auf Murashige-Skoog (MS) Agar (1%) transferiert. Die quadratischen Platten (12 x 12 cm) wurden mit Parafilm verschlossen und drei Wochen bei 23°C (8/16 h Licht-Dunkelrhythmus) gehalten. Anschließend erfolgte die Bestimmung des Frischgewichtes der *Arabidopsis-*Pflanzen. Kälte-tolerante Vertreter der Arten *Bacillus Simplex, Bacillus atrophaeus*, und *Bacillus pumilus* zeigten eine deutliche phytostimulatorische Wirkung (Abb. 4, Abb. 5).

### Beispiel 8: Topfversuch mit Kartoffelpflanzen im Gewächshaus

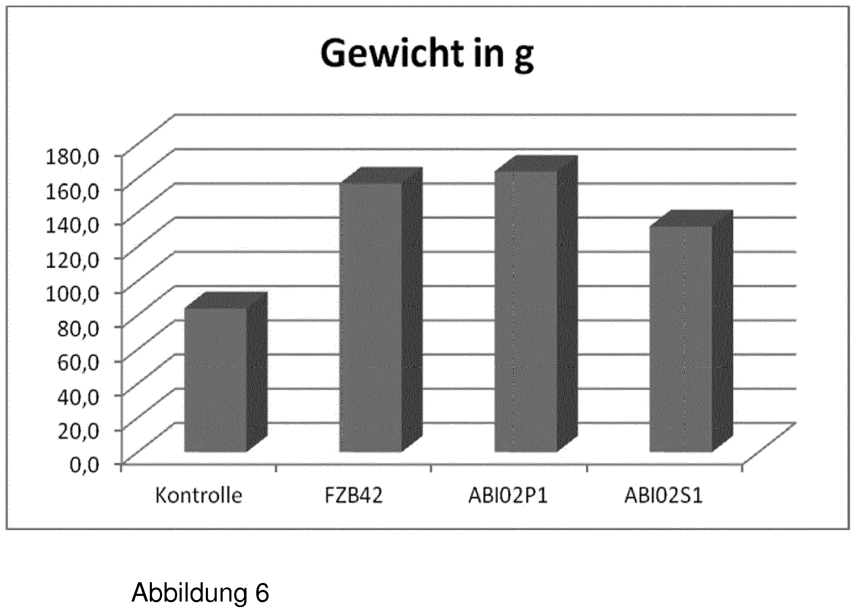

Der Versuch wurde in der Prüfstelle für Kartoffelforschung Agro Nord, D-18190 Groß-Lüsewitz als Topfversuch vom 11.07. - 11.10. 2011 durchgeführt. Die Knollen der eingesetzten Kartoffel -Versuchspflanze,Sorte "Burana", war mit Symptomen eines *Rhizoctonia solani* Befalls ("black scurf", "Pocken") belastet. Die Knollen wurden vor dem Pflanzen mit einer verdünnten Sporensuspension der Bacillus-Formulierungen "gebeizt". Die Pflanzung erfolgte nach dem Abtrocknen der Knollen. Die Ernte erfolgte drei Monate nach der Pflanzung. Die Bonitur der Kartoffelpflanzen und - knollen zeigte eine Verringerung des *Rhizoctonia*-Befalls im Vergleich zur Kontrolle um 40 -45 % bei den mit *Bacillus pumilus* ABI02P1 und *Bacillus simplex* ABI02S behandelten Varianten an. Gleichzeitig wurde eine Ertragssteigerung um 95% (ABI02P1) bzw. 57% (ABI02S) erreicht (Abb. 6). Die erzielten Ergebnisse bei der Anwendung der Kälte-toleranten Stämme sind somit mit den Ergebnissen, die bei der Anwendung von FZB42 erreicht wurden, vergleichbar. Dabei ist zu berücksichtigen, dass der Versuch im Sommer und Frühherbst durchgeführt wurde, wo die Tagesmitteltemperaturen für eine Anwendung Kälte-toleranter Bakterien keineswegs optimal sind.

### Beispiel 9: Feldversuch Kartoffeln mit B. pumilus und B. simplex

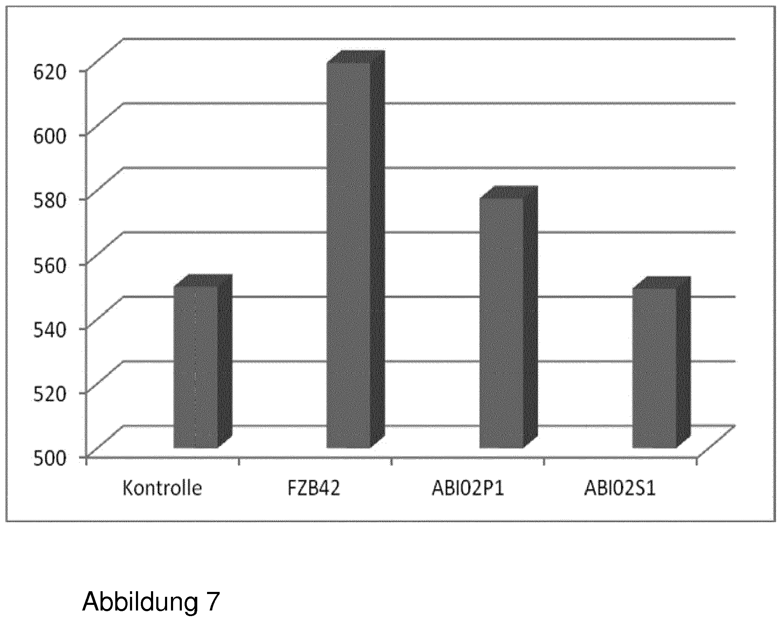

Zur Versuchsdurchführung siehe nachfolgendes Beispiel. Die Beizung der Kartoffelknollen mit dem mesophilen, pflanzenwachstumsfördernden Bakterium FZB42 führte zu einem Mehrertrag gegenüber der unbehandelten Kontrolle von 14% (Einsatzmenge: 1 L Sporensuspension/ha). Die Ertragssteigerung durch Beizung mit dem Kälte-toleranten *Bacillus pumilus* ABI02P1 (Einsatzmenge: 1L Sporensuspension/ha) betrug gegenüber der unbehandelten Kontrolle 6%. Keine Ertragssteigerung wurde bei Einsatz einer Sporensuspension des Kälte-toleranten *Bacillus simplex* ABI02S erzielt (Abb. 7). Bei der Interpretation ist zu berücksichtigen, dass der Versuch von Mai bis September durchgeführt wurde, wo die Tagesmitteltemperaturen für eine Anwendung Kälte-toleranter Bakterien keineswegs optimal sind.

### Beispiel 10: Feldversuch: Einsatz von Bacillus atrophaeus (ABI02A) führt zu Mehrertrag bei Kartoffeln

Der Feldversuch wurde von Agro Nord, Prüfstelle für Kartoffelforschung, 18190 Groß Lüsewitz, in Sanitz (Mecklenburg-Vorpommern) durchgeführt. Vorfrucht: Mais. Bodenart/-zahl IS / 35, Düngung: 140 kg N. Es kam die Kartoffelsorte "Verdi" (mittelspät - spät) zum Einsatz. Die Applikation der Sporensuspension erfolgte durch Spritzen (Parzellenspritze PL1, Düsentyp: Flachstrahldüsen TJ 800 15). Dabei wurden die eingesetzten Sporensuspensionen (2 x 10¹³ Sporen/L) in 300 L Wasser vor dem Einsatz verdünnt. Die "Beizung" erfolgte am 05.05. 2013 im Lagerhaus. Nach erfolgtem Abtrocknen der Knollen erfolgte die Aussaat am 06.05.2013 bei einer Bodentemperatur von 15,8°C.

Klimatische Bedingungen: Der Monat Mai war um 1,2 °C zu warm und um 42,5 mm (79%) zu feucht. Damit waren gute Auflaufbedingungen für die Kartoffeln gegeben. Der Monat Juni entsprach insgesamt den langjährigen Mittelwerten. Die Niederschläge fielen aber vorwiegend in Form von stärkeren Niederschlägen (13. = 26 mm; 20. = 14,3 mm; 25. = 13,5 mm) ca. 74% der Niederschlagssumme des Monats. Der einzig nennenswerte Niederschlag fiel im Juli am 3. Juli mit 28,4 mm, danach erfolgte eine sehr lange Trockenperiode, die sich bis Ende August kombiniert mit zu hohen Temperaturen fortsetzte.

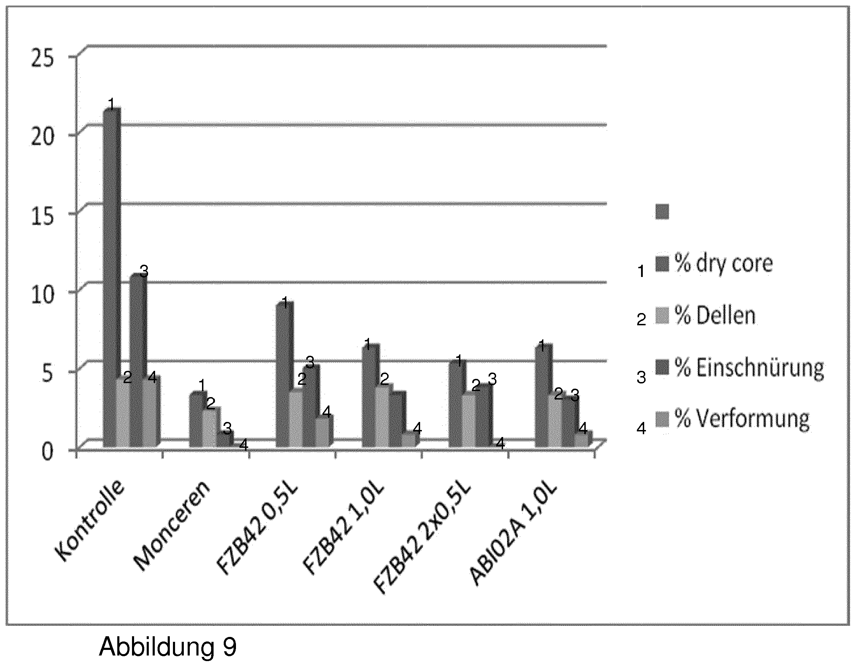

Der Versuch wurde durch das Auftreten des phytopathogen, bodenbürtigen Pilzes *Rhizoctonia solani* beeinflusst. *Rhizoctonia solani* J.G. Kühn ist die anamorphe Form von *Thanatephorus cucumeris* (A.B. Frank) Donk (teleomorph), einem Basidiomyceten aus der Familie der Agaricales. Er verursacht Ertragsverluste (Auflaufschäden, Nekrosen an Stängeln und Stolonen, viele kleine oder missförmige Knollen) sowie Qualitätsverluste (Kartoffelpocken, "Dry-core" Symptome). Die antagonistische Wirkung von *B. atrophaeus,* FZB42 und dem chemischen Fungizid Monceren Pro wurde bestimmt (Abb. 9).

Der Einsatz einer Sporensuspension (2 x 10¹⁰ cfu/ml) von *Bacillus atrophaeus* (ABI02A) in einer Konzentration von 1,0 L/ha führt zu einer Reduktion der Krankheitssymptome von "black scurf" (dry core) und einem Mehrertrag von 10%. Damit entspricht die Wirkung dieses biologischen Phytostimulator, der des chemischen Fungizids Monceren Pro (Konzentration: 1,5 L/ha).

### Beispiel 11: Feldversuch: Bestätigung: Einsatz von Bacillus atrophaeus (ABI02A) führt zu Mehrertrag bei Kartoffeln und reduziertem Befall durch Rhizoctonia solani (Kuerzinger 2014)

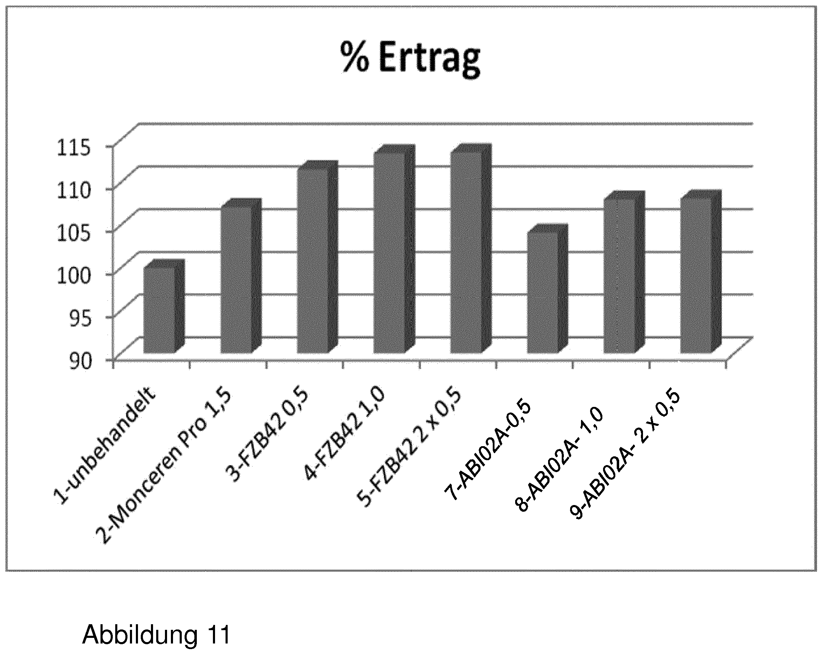

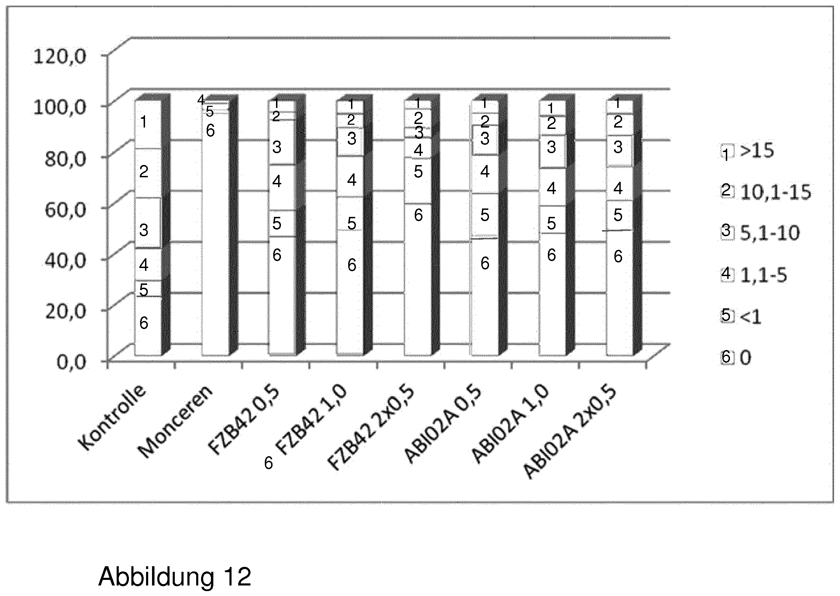

In einer zweiten Versuchsserie (Kürzinger 2014) wurden die Ergebnisse des Beispiels 10 bestätigt. Die Wirkung von *B. atrophaeus* ABI02A Sporensuspensionen war mit der des chemischen Fungizids Monceren vergleichbar (Abb. 11 und Abb. 12).

### Legende zu den Abbildungen

**Abb.1****:** Vergleich des Wachstum von *B. atrophaeus* ABI02A und dem mesophilen Stamm FZB42 bei 20°C und 25°C.

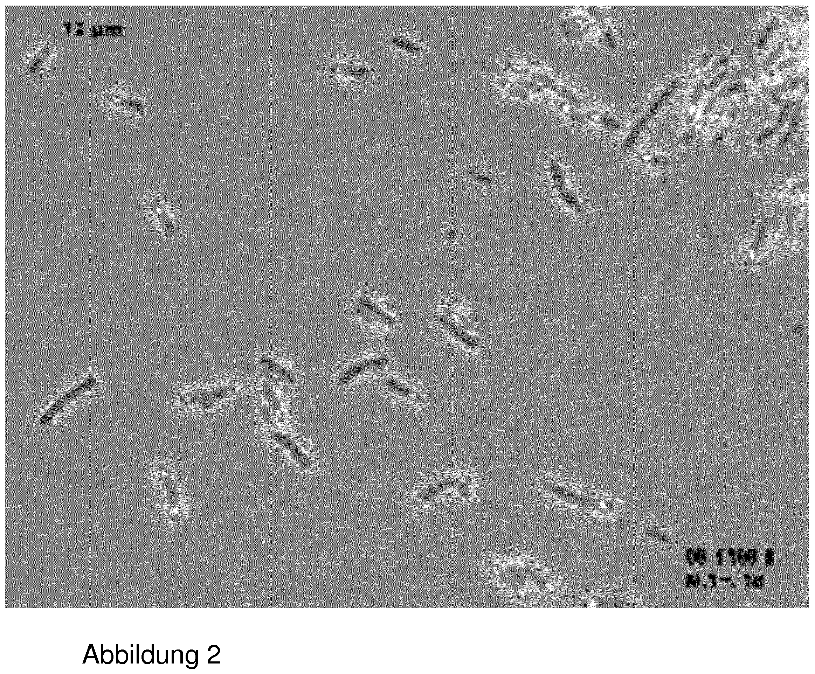

**Abb. 2****:** Phasenkontrast: *Bacillus simplex* ABI02S. Die distalen, ovalen Endosporen sind in den nicht-geschwollenen Sporenmutterzellen gut sichtbar.
**Abb. 3****:** Verbesserte Keimung von *Zea mays* nach Inkubation mit *B*. *simplex* ABI02A (untere Reihe) im Vergleich zur Kontrolle.
**Abb. 4****:** Wachstumsstimulierung (in g Frischgewicht) von *Arabidopsis thaliana* durch Sporensuspensionen von Kälte-toleranten *Bacillus*-Stämmen. Zum Vergleich: Effekt von *B.amyloliquefaciens* FZB42. Kontrolle: ohne Inokulation von *Bacillus*-Sporen. Die Säulen stellen den Mittelwert von drei unabhängigen Versuchen dar.
**Abb. 5****:** Stimulierung des Wurzelwachstums von *Arabidopsis thaliana* durch *B*. *atrophaeus* ABI02A (oben links), und *B. simplex* ABI02S (oben rechts). CK= Kontrolle ohne Inokulation mit Bakterien
**Abb. 6****:** Topf-Versuch mit Kartoffelpflanzen (Kürzinger GmbH 2011). Vergleich des Knollenertrags zur unbehandelten Kontrolle. Applikation von 1 L Sporensuspension per ha von FZB42 und *B. pumilus* ABI02P1. Die Sporensuspension von *B. simplex* ABI02S wies eine um den Faktor 5 niedrigere Konzentration auf und wurde in einer entsprechend höheren Menge (5L/ha) zu den Kartoffelpflanzen appliziert.
**Abb. 7****:** Applikation von *B. pumilus* ABI02P1 (1L/ha) und *B.simplex* ABI02S zu Kartoffelpflanzen. Einmalige Applikation durch Beizung mit der Sporensuspension (FZB42 und *Bacillus pumilus* ABI02P1: 2 x 10¹⁰ cfu/ml, *Bacillus simplex* ABI02S: 4 x 10⁹ cfu/ml) vor der Pflanzung.

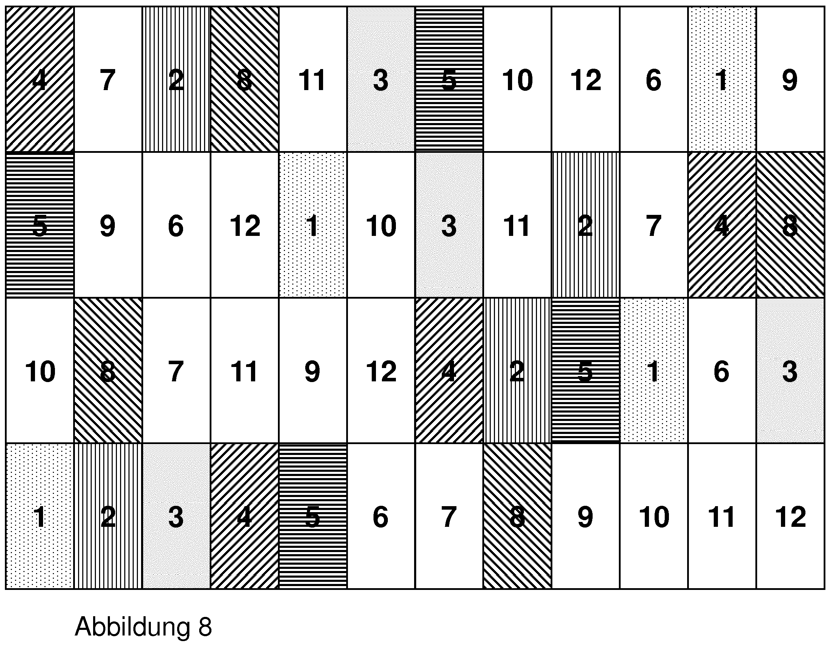

**Abb. 8** : Randomisierter Lageplan: 1 (Kontrolle, kein Zusatz), 2 chemisches Fungizid (Monceren Pro, 1,5 L/ha), 3 FZB42 (0,5 L/ha), 4 FZB42 (1,0 L/ha), 5 FZB42 (2 x 0,5 L/ha), 8 ABI02A (1 L/ha). Für jede Variante wurden vier Wiederholungen durchgeführt. Die Parzellen-Größe betrug: 6,90 m x 3,00 m = 20,70 m², Reihenabstand: 75 cm, Knollenabstand in der Reihe: 30 cm. "Beizung" der Knollen im Lagerhaus: 0.5.2013, Pflanztermin: 06.05.2013, Auflauftermin: 02.06. 2013, Ernte: 30.08.2013, Aufbereitung: 07. 10. 2013.
**Abb. 9****:** Reduktion der Krankheitssymptome ("black scurf") bei Applikation von chemischen Fungiziden (Monceren Pro) und biologischen Stimulatoren. Der Einsatz von ABI02A *'Bacillus atrophaeus'* (1,0 L/ha) führt zu einer deutlichen Reduktion der Krankheitssymptome.

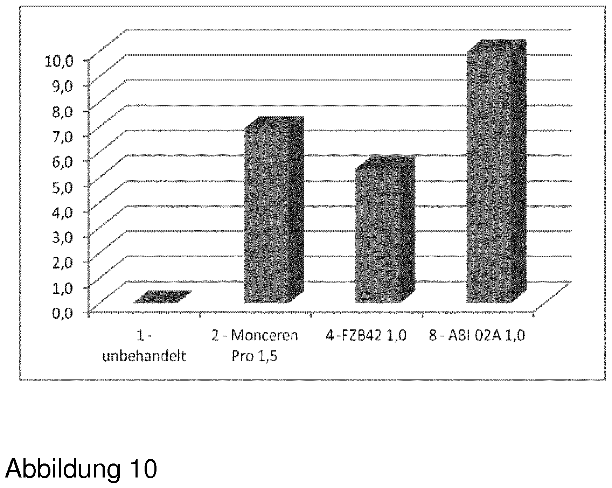

**Abb. 10****:** Einsatz von Monceren (chemisches Fungizid) und Sporensuspensionen von FZB42 und ABI02A (*Bacillus atrophaeus*) bei Kartoffeln der Sorte Verdi (Sanitz, Kürzinger 2013) führt zu Mehrertrag (%). Einsatzmengen: 1,0 = 1,0 kg/ha.
**Abb. 11****:** Die Applikation von *Bacillus atrophaeus* ABI02A führt zu einer deutlichen Ertragssteigerung, die mit der des chemischen Fungizids Monceren vergleichbar ist. Mit dem mesophilen Stamm FZB42 wurde eine etwas höhere Ertragssteigerung erzielt. Einsatzmengen der Fungizide: 1,5 L/ha (Monceren), 0,5 1,0 L/ha sowie 2x0,5L/ha bei Sporensuspensionen von FZB42 und ABI02A.
**Abb. 12****:** Gleichzeitig wurde der Sklerotienbefall (*Rhizoctonia solani*) bei Applikation von *Bacillus atrophaeus* ABI02A im Vergleich zur unbehandelten Kontrolle reduziert. Darstellung aller Befallsklassen (% Sklerotienbefall) bei Einsatz von Monceren, FZB42 und *B. atrophaeus.* B. atrophaeus führt zu einer Reduktion des Sklerotienbefalls im Vergleich zur unbehandelten Kontrolle. Befalls-Klassen: 0, <1, 1,1-5, 5,1-10, 10,1-15 und >15.

### Referenzliste

- Abd EI-Rahman, H.A., Fritze, D., Cathrin Sproer, C., Claus, D. 2002. Two novel psychrotolerant species, Bacillus psychrotolerans sp. nov. and Bacillus psychrodurans sp. nov., which contain ornithine in their cell walls. Int. J. Syst. Evol. Microbiol. 52, 2127-2133
- Bai, Y., D'Aoust, F.,Smith, D.L., Driscoll, B.T. 2002. Isolation of plant-growth-promoting Bacillus strains from soybean root nodules. Can. J. Microbiol. 48, 230-238 (2002)
- Barka, A.E., Nowk, J., Clement, C. 2006. Enhancement of chilling resistance of inoculated grapevine plantlets with plant growth promoting rhizobacteria Burkholderia phytofermans strain PsJN. Appl Environ Microbiol 72:7246-7252
- Benhamou, N., Kloepper, J.W., Quadt-Hallman, A., Tuzun, S. 1996. Induction of defense-related ultrastructural modifications in pea root tissues inoculated with endophytic bacteria. Plant Physiol. 112, 919-929
- Borriss, R. 2011. Use of plant-associated Bacillus strains as biofertilizers and biocontrol agents, In: Maheshwari DK (ed). Bacteria in agrobiology: plant growth responses. Springer, Germany, pp 41-76
- Chan, W.Y., Dietel, K., Lapa, S. et al. 2013. Draft Genome Sequence of Bacillus atrophaeus UCMB-5137, a Plant Growth-Promoting Rhizobacterium. Genome Announcement 1, e00233-13
- Erturk,Y., Cakmakci, R., Omur Duyar, O., Turan, M. 2011. The Effects of Plant Growth Promotion Rhizobacteria on Vegetative Growth and Leaf Nutrient Contents of Hazelnut Seedlings (Turkish hazelnut cv, Tombul and Sivri). Int. J. Soil Science, 6, 188-198.
- Feller, G. & Gerday C. 2003. Psychrophilic enzymes: hot topics in cold adaptation. Nature Reviews Microbiology 1, 200-208
- Gulati, A., Vyas, P., Rai, P., Kasana, R.C. 2009. Plant growth promoting and rhizosphere-competent Acinetobacter rhizosphaerae strain BIHB 723 from the cold deserts of the Himalayas. Curr.Microbiol. 58, 371-377
- Handelsman, J., Raffel, S., Mester, E.H. et al. 1990. Biological control of dampingoff of alfalfa seedlings with Bacillus-cereus UW85. Appl. Env. Microbiol. 56, 713-718
- Katiyar, V., Goel, R. 2003. Solubilization of inorganic phosphate and plant growth promotion by cold tolerant mutants of Pseudomonas fluorescens. Microbiol Res 158,163-168
- Idriss, E.E., Makarewicz, O., Farouk, A. et al. 2002. Extracellular phytase activity of Bacillus amyloliquefaciens FZB45 contributes to its plant -growth-promoting effect. Microbiology 148, 2097-2109.
- Kaushik, R., Saxena, A.K., Tilak, K.V.B.R. 2002. Can Azospirillum strains capable of growing at a suboptimal temperature perform better in field-grown-wheat rhizosphere. Biol Fertil Soils 35, 92-95
- Karlidag, H., Esitken, A., Yildrim, E. et al. 2010. Effects of plant growth promoting bacteria on yield, growth, leaf water content, membrane permeability, and ionic composition of strawberry under saline conditions. J. Plant Nutr. 34, 34-45
- Krishnamurti, S., Ruckmani, A., Pukall, R., Chakrabarti, T. 2010. Psychrobacillus gen. nov. and proposal for reclassification of Bacillus insolitus Larkin & Stokes, 1967,
- *B. psychrotolerans* Abd-EI Rahman et al., 2002 and *B. psychrodurans* Abd-EI Rahman et al., 2002 as Psychrobacillus insolitus comb. nov., Psychrobacillus psychrotolerans comb. nov. and Psychrobacillus psychrodurans comb. nov. Syst. Appl. Microbiol. 33, 367-373
- Kumar, P.K., Joshi, P., Bisht, J. K. et al. 2011. Cold-Tolerant Agriculturally Important Microorganisms. In: Plant Growth and Health Promoting Bacteria. Microbiology Monographs. Volume 18, 2011, pp 273-296.
- Löpez-Bucio, J., Campos-Cuevas, J.C., Hernändez-Calderön, E. et al. 2007. Bacillus megaterium rhizobacteria promote growth and alter root-system architecture through an auxin-and ethylene-independent signaling mechanism in Arabidopsis thaliana. Mol. Plant Microbe Interact. 20, 207-217
- Mishra, P. K., Bisht, S.C., Bisht, J. K., Bhatt, J.C. 2012. Cold-tolerant PGPRs as bioinoculants for stress management. In: Maheshwari DK (ed). Bacteria in agrobiology: Stress Management, DOI 10.1007/978-3-642-23465-1_6, # Springer-Verlag Berlin Heidelberg
- Nakamura, L. K. 1989. Taxonomic relationship of black-pigmented Bacillus subtilis strains and a proposal for Bacillus atrophaeus sp. nov. Int. J. Syst. Bacteriol. 39, 295-300.
- Prevost D, Drouin P, Laberge S, et al. 2003. Cold-adapted rhizobia for nitrogen fixation in temperate regions. Can. J. Bot. 81, 1153-1161
- Schwartz, A. R., Ortiz, I., Maymon, M. et al. 2013. Bacillus simplex - A little known PGPB with anti-fungal activity -alters pea legume root architecture and nodule morphology when coinculated with Rhizobium leguminosarum bv. viciae. Agronomy 3, 595-620
- Selvakumar, G., Kundu, S., Joshi, P. et al. 2008a. Characterization of a cold-tolerant plant growth-promoting bacterium Pantoea dispersa 1A isolated from a subalpine soil in the North Western Indian Himalayas. World J. Microbiol. Biotechnol. 24, 955-960
- Selvakumar, G., Mohan, M., Kundu, S. et al. 2008b. Cold tolerance and plant growth promotion potential of Serratia marcescens strain SRM (MTCC 8708) isolated from flowers of summer squash (Cucurbita pepo). Lett. Appl. Microbiol. 46:171-175
- Zhang, H., Prithiviraj, B., Charles, T.C. et al. 2003. Low temperature tolerant Bradyrhizobium japonicum strains allowing improved nodulation and nitrogen fixation of soybean in a short season (cool spring) area. Eur. J. Agron. 19,205-213
- Yadav Ajar Nath et al, "Cultureable diversity and functional annotation of psychrotrophic bacteria from cold desert of Leh Ladakh (India)", World journal of microbiology and Biotechnology, Rapid communications of Oxford, Oxford GB, Bd 31, Nr. 1, 5.November 2014, (201-11-05), Seiten 95-108
- Yadav Ajar Nath et al: "Prospecting cold deserts of north western Himalayas for microbial diversity and plant growth promoting attributes", Journal of Bioscience and Bioengineering, Elsevier, Amsterdam, NL, Bd. 119, Nr. 6, 6. Januar 2015 (2015-01-06), Seiten 683-693

## Patentansprüche

1. Mittel zur Stimulierung des Wachstums von Kulturpflanzen, **dadurch gekennzeichnet, dass** es den kälte-toleranten Stamm *Bacillus atrophaeus* ABI02A DSM 32019 oder ABI03 DSM 32285 oder ABI05 DSM 24918 oder eine Mischung aus zwei oder drei dieser Stämme enthält.

2. Mittel zur Stimulierung des Wachstums von Kulturpflanzen, **dadurch gekennzeichnet, dass** es einen kälte-toleranten Stamm *Bacillus simplex* ABI02S DSM 32020 oder ABI12 DSM 32283 oder eine Mischung aus beiden enthält.

3. Mittel nach Anspruch 1-2, **dadurch gekennzeichnet, dass** es einen oder eine Mischung aus den kälte-toleranten Stämmen *Bacillus atrophaeus* ABI02A DSM 32019 oder ABI03 DSM 32285 oder ABI05 DSM 24918 und einen oder eine Mischung der kälte-toleranten Stämme *Bacillus simplex* ABI02S DSM 32020 oder ABI12 DSM 32283 enthält.

4. Mittel nach Anspruch 1 - 3, **dadurch gekennzeichnet, dass** es als flüssige Sporensuspension formuliert wird, bevorzugt mit mindestens 2x10⁹ Sporen/ml, besonders bevorzugt mit mindestens 1x10¹⁰ Sporen/ml.

5. Mittel nach Anspruch 1 - 3, **dadurch gekennzeichnet, dass** es entweder zur Saatgutbehandlung oder als Gießpräparat während der Pflanzung, bzw. als Blattsprühmittel zu einem späteren Zeitpunkt, eingesetzt wird.

6. Mittel nach Anspruch 1 - 3, **dadurch gekennzeichnet, dass** es als Trockenpräparat ("Trockenbeize") formuliert wird, bevorzugt mit mindestens 5x10⁹ Sporen/ml, besonders bevorzugt mit mindestens 2x10¹⁰ Sporen/ml.

7. Mittel nach Anspruch 1 - 3, **dadurch gekennzeichnet, dass** es neben einem oder mehrerer kälte-toleranter *Bacillus* Stämme Huminsäure enthält.

8. Verwendung der Mittel nach Anspruch 1 - 3 gemeinsam mit mesophilen, pflanzenwachstumsfördernden Bakterien der Ordnung der *Bacillales*, bevorzugt der Gattung *Bacillus* oder *Paenibacillus*, besonders bevorzugt der Art *Bacillus amyloliquefaciens* ssp. *plantarum*

9. Verwendung der Mittel nach Anspruch 1 - 3 gemeinsam mit dem pflanzenwachstumsfördernden Pilz *Trichoderma sp.*

## Claims

1. Composition for stimulating the growth of cultivated plants, **characterized in that** it contains the cold-tolerant strain Bacillus atrophaeus ABI02A DSM 32019 or ABI03 DSM 32285 or ABI05 DSM 24918 or a mixture of two or three of these strains.

2. Composition for stimulating the growth of cultivated plants, **characterized in that** it comprises a cold-tolerant strain Bacillus simplex ABI02S DSM 32020 or ABI12 DSM 32283 or a mixture of both.

3. Composition of claim 1 - 2, **characterized in that** it contains one or a mixture of the cold-tolerant strains Bacillus atrophaeus ABI02A DSM 32019 or ABI03 DSM 32285 or ABI05 DSM 24918 and one or a mixture of the cold-tolerant strains Bacillus simplex ABI02S DSM 32020 or ABI12 DSM 32283.

4. Composition according to claim 1 - 3, **characterized in that** it is formulated as a liquid spore suspension, preferably with at least 2x10⁹ spores/ml, particularly preferably with at least 1x10¹⁰ spores/ml.

5. Composition according to claim 1-3, **characterized in that** it is used either for seed treatment or as a watering preparation during planting, or as a foliar spraying agent at a later time.

6. Composition according to claim 1 - 3, **characterized in that** it is formulated as a dry preparation ("dry stain"), preferably with at least 5x10⁹ spores/ml, particularly preferably with at least 2x10¹⁰ spores/ml.

7. Composition according to claim 1-3, **characterized in that** it contains humic acid in addition to one or more cold-tolerant Bacillus strains.

8. Use of the composition according to claim 1-3 together with mesophilic, plant growth-promoting bacteria of the order of Bacillales, preferably of the genus Bacillus or Paenibacillus, particularly preferably of the species Bacillus amyloliquefaciens ssp. plantarum

9. Use of the compositions according to claims 1 - 3 together with the plant growth promoting fungus Trichoderma sp.

## Revendications

1. Agent stimulant la croissance des plantes cultivées, **caractérisé par le fait qu'**il contient la souche résistante au froid *Bacillus atrophaeus* ABI02A DSM 32019 ou ABI03 DSM 32285 ou ABI05 DSM 24918, ou bien un mélange constitué de deux ou trois de ces souches.

2. Agent stimulant la croissance des plantes cultivées, **caractérisé par le fait qu'**il contient une souche résistante au froid *Bacillus simplex* ABI02S DSM 32020 ou ABI12 DSM 32283, ou bien un mélange constitué de ces deux souches.

3. Agent au sens des revendications 1 - 2, **caractérisé par le fait qu'**il contient une souche résistante au froid *Bacillus atrophaeus* ABI02A DSM 32019 ou ABI03 DSM 32285 ou ABI05 DSM 24918, ou un mélange constitué de ces souches, et une souche résistante au froid *Bacillus simplex* ABI02S DSM 32020 ou ABI12 DSM 32283, ou un mélange constitué de ces souches.

4. Agent au sens des revendications 1 - 3, **caractérisé par le fait qu'**il est formulé sous forme de suspension de spores liquide contenant de préférence au moins 2x10⁹ spores/ml ou, dans l'idéal, au moins 1x10¹⁰ spores/ml.

5. Agent au sens des revendications 1 - 3, **caractérisé par le fait qu'**il est utilisé pour le traitement des semences, pour l'arrosage lors de la plantation ou pour la vaporisation des feuilles à un stade ultérieur.

6. Agent au sens des revendications 1 - 3, **caractérisé par le fait qu'**il est formulé sous forme de préparation sèche (pour le traitement des semences) contenant de préférence au moins 5x10⁹ spores/ml ou, dans l'idéal, au moins 2x10¹⁰ spores/ml.

7. Agent au sens des revendications 1 - 3, **caractérisé par le fait qu'**il contient une ou plusieurs souches résistantes au froid *Bacillus* ainsi que de l'acide humique.

8. Utilisation des agents au sens des revendications 1 - 3 avec des bactéries mésophiles stimulant la croissance des plantes et appartenant à l'ordre des *Bacillales,* de préférence au genre *Bacillus* ou *Paenibacillus* et, dans l'idéal, à l'espèce *Bacillus amyloliquefaciens,* sous-espèce *plantarum.*

9. Utilisation des agents au sens des revendications 1 - 3 avec le champignon stimulant la croissance des plantes *Trichoderma sp.*
